(19) Europäisches Patentamt
European Patent Office
Office européen des brevets

(11) Numéro de publication: **0 057 033**
**B1**

(12) # FASCICULE DE BREVET EUROPEEN

(45) Date de publication du fascicule du brevet: **21.11.84**

(51) Int. Cl.³: **A 61 F 1/00**, C 08 J 7/18

(21) Numéro de dépôt: **82200046.9**

(22) Date de dépôt: **15.01.82**

(54) **Prothèse destinée à être insérée dans des tissus vivants, en particulier prothèse mammaire et procédé de fabrication.**

(30) Priorité: **26.01.81 FR 8101553**

(43) Date de publication de la demande:
**04.08.82 Bulletin 82/31**

(45) Mention de la délivrance du brevet:
**21.11.84 Bulletin 84/47**

(84) Etats contractants désignés:
**AT BE CH DE GB IT LI LU NL SE**

(56) Documents cités:
**FR-A-1 526 934**
**FR-A-2 140 560**
**FR-A-2 208 775**
**FR-A-2 251 423**
**FR-A-2 352 851**
**GB-A-860 327**
**GB-A-2 051 844**

(73) Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Feurer, Bernard, 31 rue des Tourterelles, F-31650 Saint Orens de Gameville (FR)**
Inventeur: **Chavoin, Jean-Pierre, 17 avenue Honoré Serres, F-31400 Toulouse (FR)**
Inventeur: **Baronet, Philippe, 32 rue Alsace Lorraine, F-31000 Toulouse (FR)**
Inventeur: **Morucci, Jean-Pierre, 75 rue du Cagire, F-31100 Toulouse (FR)**

(74) Mandataire: **Barre, Philippe, Cabinet Barre, Gatti, Laforgue 95, rue des Amidonniers, F-31069 Toulouse Cedex (FR)**

EP 0 057 033 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne une prothèse destinée à être insérée dans des tissus vivants, en particulier une prothèse mammaire du type comprenant une enveloppe en silicone de qualité médicale et un gel ou une huile de même type, contenu dans ladite enveloppe. Elle s'étend à un procédé de fabrication de ladite prothèse.

On sait que, dans le domaine de la chirurgie plastique et esthétique, la mise en place de prothèses mammaires est devenue courante, soit pour réaliser une reconstruction mammaire après amputation ou mastectomie sous-cutanée, soit pour effectuer des corrections d'hypoplasies, d'aplasies ou d'agénésies mammaires congénitales. Les prothèses mammaires les plus modernes sont constituées par une enveloppe de silicone remplie d'un gel ou d'une huile de silicone.

Toutefois, on a pu constater dans près de 70% des cas, que la présence de ces prothèses inserées dans les tissus vivants entraînait la formation de coques fibreuses rétractiles qui engendraient une perte de souplesse du sein au bout de quelques mois.

Plusieurs solutions ont été proposées pour pallier cet inconvénient, mais aucune n'est satisfaisante.

En particulier, on a proposé d'enrober la prothèse au moyen d'une couche d'un matériau différent tel que mousse de polyuréthane (prothèses d'Ashley) mais l'épaisseur de l'enveloppe obtenue est notablement plus importante et cette enveloppe est perceptible au palper du sein et également à la vue, les bords de celle-ci formant un relief inesthétique; de plus, le polyuréthane est un matériau susceptible d'evoluer et de se dégrader au cours du temps, de sorte que son utilisation dans ce type d'application paraît peut indiquée.

Par ailleurs, on a essayé de remplir les enveloppes des prothèses de sérum physiologique, mais on a pu constater que les prothèses avaient, à la longue, tendance à perdre du volume et à emmagasiner des bulles d'air. En outre, dans le brevet US n° 4 138 382, on a propose de remplacer le gel ou l'huile de silicone par un nouveau gel de nature hydrophile, mais on ne profite pas alors des qualités bien connues des gels de silicone (inertie biologique, souplesse se rapprochant de celle des tissus mammaires, produit courant relativement peu onéreux . . .).

La présente invention se propose d'indiquer une solution satisfaisante au problème ci-dessus posé, permettant de limiter considérablement, les réactions de formation de fibres des tissus autour des prothèses et de supprimer le caractère rétractile des coques fibreuses éventuellement formées, et ce, sans présenter les défauts des solutions connues sus-évoquées.

Un objectif de l'invention est en particulier d'indiquer un procédé de fabrication de prothèse, fournissant une prothèse correctement tolérée par les tissus vivants dans lesquels elle est insérée, en vue de former avec ces tissus un organe gardant ses caractéristiques initiales au cours du temps.

A cet effet, le procédé de fabrication visé par l'invention s'applique à toute prothèse du type comprenant une enveloppe en un polymère hydrophobe (notamment en silicone) contenant un gel ou une huile polymère hydrophobe (notamment du même type); le procédé conforme à la présente invention est caracterisé en ce qu'on fait subir à au moins une des faces de l'enveloppe un traitement de surface adapté pour rendre la ou lesdites faces hydrophiles.

De préférence, ce traitement de surface est appliqué aux deux faces de l'enveloppe en vue de les rendre toutes deux hydrophiles.

Une telle modification en surface des faces interne et externe de l'enveloppe de prothèse, engendre, d'une part, une modification du comportement de celle-ci à l'égard du gel ou huile qu'elle contient, d'autre part, une modification de son comportement à l'égard des tissus externes.

En premier lieu, le caractère hydrophile des faces de l'enveloppe supprime ou réduit considérablement la diffusion du gel ou huile interne à travers l'enveloppe, et ce, malgré la faible épaisseur de celle-ci et les faibles masses moléculaires des gels ou huiles de silicone ou analogue; cette absence de diffusion provient d'une réaction de répulsion qui se produit à l'interface entre un gel ou liquide hydrophobe et une surface d'enveloppe devenue hydrophile. Or, la diffusion des particules du gel ou d'huile à travers la paroi de l'enveloppe vers les tissus est une des causes essentielles des réactions de formation de fibres de ces derniers, entraînant la formation de la coque fibreuse rétractile et dure, déjà mentionnée. On limite ainsi la réaction à corps étranger et la tendance rétractile de la coque est supprimée, sinon la coque elle-même: le sein garde alors dans le temps une souplesse sensiblement identique à sa souplesse initiale.

En outre, on a pu constater que le caractère hydrophile conféré à la face externe de l'enveloppe améliore notablement la compatibilité de cette enveloppe avec les tissus et limitait, à l'interface, les phénomènes d'exclusion.

Le traitement de surface sus-évoqué peut consister en tout procédé connu pour rendre hydrophile la surface d'un polymère hydrophobe, soit en modifiant les caractéristiques de cette surface, soit en effectuant un dépôt mince approprié sur cette surface. Il est bien entendu que plusieurs types de procédés de traitement permettant de rendre hydrophile une surface donnée sont connus en eux-mêmes, mais ces procédés n'ont jamais été appliqués au traitement d'une prothèse appelée à être insérée dans des tissus vivants. L'invention revendiquée vise donc le traitement d'une telle prothèse afin de limiter les réactions de formation de fibres des tissus vivants et de réduire le caractère rétractile des éventuelles coques formées par ceux-ci, grâce aux déroulements des phénomènes

sus-évoqués (suppression de la diffusion du gel hydrophobe contenu dans l'enveloppe et limitation des réactions d'exclusion à l'interface).

Selon un premier mode de mise-en-œuvre, le traitement peut consister en un bombardement ionique de la surface de l'enveloppe de la prothèse au moyen d'un faisceau d'ions, adapté pour transformer les sites hydrophobes de celle-ci en sites hydrophiles.

Le faisceau est de préférence un faisceau d'ions positifs, obtenu en soumettant un gaz du groupe: azote, oxygène, vapeur d'eau, gaz carbonique ... à un champ électrique. De tels traitements de surface sont connus en soi et entraînent des modifications physico-chimiques en surface des polymères leur conférant un caractère hydrophile marqué.

Selon un autre mode de mise-en-œuvre, le traitement de la prothèse consiste à disposer l'enveloppe dans une atmosphère gazeuse et à créer au moyen d'une décharge électrique un plasma adapté pour transformer les sites hydrophobes de la surface en sites hydrophiles; le processus de transformation est analogue au cas précédent. Les gaz utilisés peuvent être du groupe ci-dessus mentionné.

Dans le cas où le polymére de l'enveloppe possède des fonctions réactives, un autre mode de mise en œuvre du procédé de l'invention consiste à greffer sur ces fonctions réactives un ou des composés hydrophiles ou à fonction polaire. Par exemple, pour une enveloppe en silicone, il est possible de greffer un ou des composés hydrophiles sur les liaisons libres silicone-hydrogène; cette greffe peut s'effectuer par une réaction sur les liaisons Si—H de composés hydrophiles comportant une double liaison réactive en présence d'un catalyseur au platine

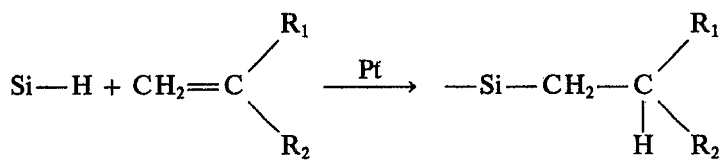


Un autre processus consiste à créer en surface du polymère des sites actifs puis à effectuer sur ces sites la greffe ci-dessus évoquée du ou des composés hydrophiles ou à fonction polaire. Par exemple, dans le cas du silicone, une irradiation par un rayonnement Gamma ou Ultraviolet de faible longueur d'onde permet d'activer les liaisons silicone $-CH_3$ en créant, soit des liaisons identiques excitées, soit des radicaux libres silicone $-CH_2$, qui permettront de réaliser la greffe des composés hydrophiles.

Ce ou ces derniers seront en particulier des composés du groupe: hydroxy éthyl méthacrylate, hydroxy propyl méthacrylate, hydroxy éthyl acrylate, acide méthacrylique, acide acrylique, acrylonitrile, alcool vinylique, alcool allylique, vinyl pyridine, vinyl pyrrolidone, méthacrylamide.

Selon un autre mode de mise en œuvre, le traitement de surface peut consister à déposer un film mince de polymère sur la surface à partir d'un monomère vaporisé dans une enceinte sous vide contenant l'enveloppe. Comme cela est connu en soi, la polymérisation de ce monomère vaporisé peut être obtenue soit par l'intermédiaire d'un plasma engendré par une décharge électrique, soit par une irradiation appropriée (ultraviolet par exemple). Les monomères utilisés peuvent être choisis dans le groupe cité précédemment.

Le traitement de surface peut également consister à déposer par tout moyen connu sur les faces de l'enveloppe, un film mince d'un matériau possedant des sites hydrophiles (matériau de nature hydrophile ou matériau préalablement traité pour greffer sur celui-ci des sites hydrophiles).

L'invention s'applique en particulier pour réaliser des prothèses mammaires dont l'enveloppe et le gel ou l'huile contenu dans celle-ci sont à base de silicone.

L'exemple qui suit est destiné à illustrer les résultats obtenus.

## Exemple

L'enveloppe choisie est une enveloppe en silicone réticulé du type »Rhône Poulenc RTV 141« de 23/100 mm d'épaisseur. De façon classique, cette enveloppe présente une partie avant de forme à peu près hémisphérique et une partie arrière à peu près plane, dotée d'une ouverture centrale. On effectue d'abord le traitement de surface ci-après décrit de la face externe de l'enveloppe, puis on retourne celle-ci grâce à la présence de son ouverture et on effectue le traitement de surface de l'autre face ainsi disposée à l'extérieur.

Chacun de ces traitements de surface est réalisé dans une enceinte sous une pression de $2.10^{-4}$ millibar; dans cette enceinte est disposée une source de type classique fournissant en l'exemple des ions oxygénes positifs avec une intensité de courant de cathode de 31 micro-Ampères et une tension d'accélération de 5,2 kilovolts. Le faisceau étroit obtenu est sensiblement homocinétique et est amené à balayer la surface de l'enveloppe avec une incidence d'environ 45°; toute la surface de l'enveloppe est traitée par va-et-vient successifs, rotations du plateau porte-enveloppe et basculement de l'enveloppe. La durée de traitement est de 15 minutes en toute zone (la distance de la source

à l'échantillon étant d'environ 6 cm).

Les deux faces de l'enveloppe sont ainsi traitées de même que les deux faces d'une pièce circulaire du même matériau destinée à obturer l'ouverture de ladite enveloppe.

Pour parfaire le traitement d'hydrophilisation, on amène l'enveloppe et la pièce circulaire à séjourner 3 heures dans de l'eau à 70° C.

A l'issu de ce traitement, on constate que les angles de mouillage à l'eau de la surface sont passés de 105° à 50°, ce qui montre que le caractère initial hydrophobe a disparu et a été remplacé par un caractère hydrophile marqué.

Après remise à l'endroit, on remplit l'enveloppe d'huile du type »Rhodorsil 47V5« et on la ferme par collage de la pièce circulaire avec une colle au silicone. Notons que celle huile est très fluide (viscosité: 5 cSt) et n'est en fait pas utilisé en pratique pour les prothèses mammaires car sa diffusion élevée entraînerait, dans le cas des prothèses connues, une formation de fibres des tissus extrêmement rapide et tout-à-fait inacceptable. Cette huile a été utilisée dans cet exemple pour illustrer les résultats obtenus.

Au bout de 24 heures, la diffusion d'huile à travers l'enveloppe a été mesurée par chromatographie de perméation sur gel (G. P. C.); elle a montré une diffusion nulle.

Une mesure comparative avec une enveloppe similaire non traitée donne au bout de 24 heures une diffusion notable.

De nouvelle mesures au bout de 96 heures montrent que la diffusion de l'enveloppe traitée est 16 fois moindre que celle de l'enveloppe non traitée.

La même enveloppe est ensuite ouverte, nettoyée et remplie d'un gel de silicone de forte viscosité, de type utilisé habituellement dans les prothèses mammaires. Au bout de 3 semaines, aucune diffusion n'a été constatée par mesure chromatographique.

Des expérimentations avec des gels de masses moléculaires différentes ou polydisperses ont montré que la diffusion était moindre ou nulle pour les molécules ayant les masses les plus élevées.

Bien entendu, l'invention n'est pas limitée aux termes de la description précédentes, mais en comprend les variantes; en particulier, l'enveloppe en silicone traitée peut être associée à un revêtement intérieur pour fournir un élément multi-couches; la face intérieure traitée de l'enveloppe en silicone constitue alors une barrière à l'égard de la diffusion du gel ou de l'huile interne.

**Revendications**

1. Procédé de fabrication d'une prothèse destinée à être insérée dans des tissus vivants, cette prothèse étant de nature à limiter les réactions de formation de fibres des tissus et à supprimer le caractère rétractile des coques fibreuses éventuellement formées, ledit procédé consistant à réaliser une enveloppe en un polymère hydrophobe notamment en silicone, et à remplir ladite enveloppe au moyen d'un gel ou d'une huile polymère hydrophobe, notamment de même nature que l'enveloppe caracterisé en ce qu'avant de remplir l'enveloppe on fait subir à au moins une des faces de l'enveloppe un traitement de surface adapté pour rendre la ou lesdites faces hydrophiles.

2. Prothèse destinée à être insérée dans des tissus vivants et de nature à limiter les réactions de formation de fibres des tissus et à supprimer le caractère rétractile des coques fibreuses éventuellement formées, ladite prothèse comprenant une enveloppe en un polymère hydrophobe, en particulier à base de silicone, et un gel ou une huile polymère hydrophobe, en particulier à base de silicone, contenu dans ladite enveloppe caracterisé en ce que ladite enveloppe comporte au moins une face traitée pour présenter un caractère hydrophile en surface.

3. Procédé selon la revendication 1, caractérisé en ce que l'on fait subir le traitement de surface aux deux faces de l'enveloppe en vue de rendre ces deux faces hydrophiles.

4. Prothèse selon la revendication 2, caractérisée en ce que l'enveloppe a ses deux faces traitées pour présenter un caractère hydrophile.

5. Procédé selon l'une des revendications 1 ou 3, dans lequel le traitement de surface de l'enveloppe est réalisé par un bombardement ionique de la surface au moyen d'un faisceau d'ions, adapté pour transformer les sites hydrophobes de celle-ci en sites hydrophiles.

6. Procédé selon l'une des revendications 1 ou 3, dans lequel le traitement de surface de l'enveloppe est réalisé en disposant celle-ci dans une atmosphère gazeuse et en créant au moyen d'une décharge électrique un plasma, adapté pour transformer les sites hydrophobes de la surface en sites hydrophiles.

7. Procédé selon l'une des revendications 1 ou 3, dans lequel le traitement de surface de l'enveloppe consiste à greffer sur des fonctions réactives du polymère de l'enveloppe un ou des composés hydrophiles ou à fonction polaire.

8. Procédé selon l'une des revendications 1 ou 3, dans lequel le traitement de surface de l'enveloppe consiste à créer des sites actifs en surface de celle-ci puis à greffer sur ces sites un ou des composés hydrophiles ou à fonction polaire.

9. Procédé selon l'une des revendications 1 ou 3, dans lequel le traitement de surface de l'enveloppe consiste à déposer un film mince de polymère obtenu à partir d'un composé monomère vaporisé.

10. Procédé selon l'une des revendications 7, 8 ou 9, dans lequel l'enveloppe est en silicone et le composé greffé ou le composé déposé est un composé ou un mélange de composés du groupe: hydroxyéthyl-méthacrylate, hydroxy propyl méthacrylate, hydroxy éthyl acrylate, acide méthacrylique, acide acrylique, acrylonitrile, alcool vinylique, alcool allylique, vinyl pyridine, vinyl pyrrolidone, méthacrylamide.

11. Procédé selon la revendication 3, caractérisé en ce qu'on effectue d'abord le traitement de surface d'une face de l'enveloppe, on retourne ensuite ladite enveloppe grâce à une ouverture dont celle-ci est pourvue, on effectue le traitement de surface de l'autre face ainsi disposée à l'extérieur, et, le cas échéant après remise à l'endroit, on remplit l'enveloppe de gel ou d'huile par son ouverture et on obture ladite ouverture avec une pièce du même matériau que l'enveloppe, ayant subi le même traitement de surface sur ses deux faces.

12. Prothèse mammaire selon l'une des revendications 2 ou 4, dont l'enveloppe de même que le gel ou l'huile contenu dans celle-ci sont à base de silicone.

**Patentansprüche**

1. Verfahren zur Herstellung einer zum Implantieren in lebende Gewebe bestimmten Prothese, wobei diese Prothese so beschaffen ist, daß sie die Faserbildungsreaktionen in den Geweben begrenzt und die Einziehbarkeit der unter Umständen gebildeten Faserschalen ausschaltet, wobei besagtes Verfahren in der Herstellung einer Hülle aus einem hydrophoben Polymer, insbesondere Silikon, besteht, dadurch gekennzeichnet, daß, ehe die Hülle gefüllt wird, mindestens eine der Hüllenflächen einer zur hydrophilen Gestaltung der besagten Fläche oder Flächen geeigneten Oberflächenbehandlung unterzogen und besagte Hülle mit einem hydrophoben polymeren Gel oder Öl, insbesondere von einem dem der Hülle gleichen Typ, gefüllt wird.

2. Zum Implantieren in lebende Gewebe bestimmte Prothese, welche so beschaffen ist, daß sie die Faserbildungsreaktionen in den Geweben begrenzt und die Einziehbarkeit der unter Umständen gebildeten Faserschalen ausschaltet, wobei besagte Prothese eine Hülle aus einem hydrophoben Polymer, insbesondere auf Silikon-Grundlage, umfaßt, dadurch gekennzeichnet, daß besagte Hülle mindestens eine Fläche, welche so behandelt ist, daß sie eine hydrophile Oberflächen-Natur aufweist, und ein in besagter Hülle enthaltenes hydrophobes Gel oder Öl, insbesondere auf Silikon-Grundlage, umfaßt.

3. Verfahren nach Anspruch 1, dadurch gekennzeichnet, daß man die beiden Hüllenflächen der Oberflächenbehandlung unterzieht, um diese beiden Flächen hydrophil zu machen.

4. Prothese nach Anspruch 2, dadurch gekennzeichnet, daß beide Flächen der Hülle behandelt werden, um eine hydrophile Natur aufzuweisen.

5. Verfahren nach einem der Ansprüche 1 oder 3, bei welchem die Oberflächenbehandlung der Hülle durch Ionen-Bombardement der Oberfläche mittels eines Ionen-Bündels durchgeführt wird, welches dazu geeignet ist, die hydrophoben Stellen derselben in hydrophile Stellen umzuwandeln.

6. Verfahren nach einem der Ansprüche 1 oder 3, bei welchem die Oberflächenbehandlung der Hülle derart durchgeführt wird, daß diese in eine gasförmige Atmosphäre gebracht und mittels einer elektrischen Entladung ein zur Umwandlung der hydrophoben Stellen der Oberfläche in hydrophile Stellen befähigtes Plasma erzeugt wird.

7. Verfahren nach einem der Ansprüche 1 oder 3, bei welchem die Oberflächenbehandlung der Hülle darin besteht, daß man auf reaktionsfähige Gruppen des Polymers der Hülle eine oder mehrere hydrophile Verbindungen oder solche mit Polar-Gruppen aufpfropft.

8. Verfahren nach einem der Ansprüche 1 oder 3, bei welchem die Oberflächenbehandlung der Hülle darin besteht, daß man aktive Stellen an der Oberfläche derselben erzeugt und dann eine oder mehrere hydrophile Verbindungen oder solche mit Polar-Gruppen auf diese Stellen aufpfropft.

9. Verfahren nach einem der Ansprüche 1 oder 3, bei welchem die Oberflächenbehandlung der Hülle darin besteht, daß man einen dünnen Film eines aus einer verdampften monomeren Verbindung erhaltenen Polymers abscheidet.

10. Verfahren nach einem der Ansprüche 7, 8 oder 9, bei welchem die Hülle aus Silikon besteht und die aufgepfropfte Verbindung oder die abgeschiedene Verbindung eine Verbindung oder eine Mischung aus Verbindungen der Gruppe: Hydroxyäthylmethacrylat, Hydroxypropylmethacrylat, Hydroxyäthylacrylat, Methacrylsäure, Acrylsäure, Acrylonitril, Vinylalkohol, Allylalkohol, Vinylpyridin, Vinylpyrrolidon und Methacrylamid ist.

11. Verfahren nach Anspruch 3, dadurch gekennzeichnet, daß man zunächst die Oberflächenbehandlung an einer Hüllenfläche vornimmt, dann besagte Hülle mit Hilfe einer Öffnung, mit der diese versehen ist, wendet, die Oberflächenbehandlung an der anderen, somit nach außen liegenden Fläche vornimmt und, gegebenenfalls nach dem Einsetzen die Hülle mit Gel oder Öl durch ihre Öffnung füllt und besagte Öffnung mit einem Stück aus dem gleichen Stoff wie die Hülle abdichtet, welches der gleichen Oberflächenbehandlung auf seinen beiden Flächen unterzogen worden ist.

12. Brustprothese nach einem der Ansprüche 2 oder 4, deren Hülle sowie das in dieser enthaltene Gel oder Öl eine Silikon-Grundlage aufweisen.

## Claims

1. Method for the manufacture of a prosthesis for insertion into living tissues, this prosthesis being such as to limit the fibre-forming reactions in the tissues and to overcome the retractability of the fibrous shells that may be formed, said method consisting in producing an envelope from a hydrophobic polymer, particularly silicone, characterised in that, prior to filling the envelope, at least one of the faces of the envelope is subjected to a surface treatment, suitable for making said face or faces hydrophilic, and said envelope is filled with a hydrophobic polymeric gel or oil, particularly of the same type as the envelope.

2. Prosthesis for insertion into living tissues, being such as to limit the fibre-forming reactions in the tissues and to overcome the retractability of the fibrous shells that may be formed, said prosthesis comprising an envelope, made from a hydrophobic polymer, particularly silicone-based, characterised in that said envelope comprises at least one face which has been treated so as to exhibit a hydrophilic surface nature, and a hydrophobic polymeric gel or oil, particularly silicone-based, contained in said envelope.

3. Method according to claim 1, characterised in that the two faces of the envelope are subjected to the surface treatment, for making these two faces hydrophilic.

4. Prosthesis according to claim 2, characterised in that the envelope has both of its faces treated so as to exhibit a hydrophilic nature.

5. Method according to either claim 1 or 3, in which the surface treatment of the envelope is effected by ion bombardment of the surface by means of an ion bundle, capable of converting the hydrophobic sites of the surface into hydrophilic sites.

6. Method according to either claim 1 or 3, in which the surface treatment of the envelope is effected by placing the latter in a gaseous atmosphere and by generating by means of an electric discharge a plasma, capable of converting the hydrophobic sites of the surface into hydrophilic sites.

7. Method according to either claim 1 or 3, in which the surface treatment of the envelope consists in grafting on the reactive groups of the polymer of the envelope one or more hydrophilic compounds or compounds having polar groups.

8. Method according to either claim 1 or 3, in which the surface treatment of the envelope consists in creating active sites in the surface of the latter and then grafting on these sites one or more hydrophilic compounds or compounds having polar groups.

9. Method according to either claim 1 or 3, in which the surface treatment of the envelope consists in depositing a thin polymer film, obtained from a vaporised monomeric compound.

10. Method according to one of the claims 7, 8 or 9, in which the envelope is made of silicone and the grafted compound or deposited compound is a compound or mixture of compounds of the group: hydroxyethyl methacrylate, hydroxypropyl methacrylate, hydroxyethyl acrylate, methacrylic acid, acrylic acid, acrylonitrile, vinyl alcohol, allyl alcohol, vinyl pyridine, vinyl pyrrolidone and methacrylamide.

11. Method according to claim 3, characterised in that first the surface treatment is carried out on one face of the envelope, then said envelope is reversed with the aid of an aperture with which the latter is provided, the surface treatment is carried out on the other face thus turned outwards and, if appropriate after putting it in place, the envelope is filled with gel or oil through its aperture and said aperture is sealed with a piece of the same material as the envelope, having undergone the same surface treatment on its two faces.

12. Mammary prosthesis according to either claim 2 or 4, the envelope of which and the gel or oil, contained in the latter, are silicone-based.